# EUROPÄISCHE PATENTSCHRIFT

(11) **EP 2 828 663 B1**
(45) Veröffentlichungstag und Bekanntmachung des Hinweises auf die Patenterteilung: **26.09.2018**
(21) Anmeldenummer: 13720803.9
(22) Anmeldetag: 22.03.2013
(51) Int. Cl.: G01N 33/569

(54) **NEUE MSC-OBERFLÄCHENMARKER**
NOVEL MSC SURFACE MARKER
NOUVEAU MARQUEUR DE SURFACE DES CELLULES SOUCHES MÉSENCHYMATEUSES (CSM)

(30) Priorität: 23.03.2012 DE 102012102532
(43) Veröffentlichungstag der Anmeldung: 28.01.2015
(73) Patentinhaber: Eberhard-Karls-Universität Tübingen Universitätsklinikum, 72076 Tübingen (DE)
(72) Erfinder: BUEHRING, Hans-Joerg, 72076 Tübingen (DE); GRIMM, Sabrina, 72108 Rottenburg a.N. (DE); CERABONA, Flavianna, 72406 Bissingen (DE)
(74) Vertreter: Witte, Weller & Partner Patentanwälte mbB
(86) Internationale Anmeldenummer: PCT/EP2013/056033
(87) Internationale Veröffentlichungsnummer: WO 2013/139952

(56) Entgegenhaltungen:
- ES-A1- 2 370 794
- HANS-JÖRG BÜHRING ET AL: "Phenotypic Characterization of Distinct Human Bone Marrow-Derived MSC Subsets", ANNALS OF THE NEW YORK ACADEMY OF SCIENCES, Bd. 1176, Nr. 1, 1. September 2009 (2009-09-01), Seiten 124-134, XP55026649, ISSN: 0077-8923, DOI: 10.1111/j.1749-6632.2009.04564.x

## Beschreibung

Die vorliegende Erfindung betrifft die *in vitro* Isolierung und/oder Identifizierung von hochreinen mesenchymalen Stammzellen mittels neuer Zelloberflächenmarker, sowie die Verwendung dieser Zelloberflächenmarker.

Mesenchymale Stammzellen (MSC), auch mesenchymale stromale Zellen genannt, sind multipotente Zellen, die die Fähigkeit besitzen, unter geeigneten *in vitro-* und *in vivo*-Bedingungen in verschiedene mesenchymale Gewebe auszudifferenzieren. So können sie bspw. in Osteocyten, Chondrocyten, Adipozyten, Myocyten differenzieren, und Knochen-, Knorpel-, Fett-, und Muskelgewebe bilden. Darüber hinaus können sie sich aber auch in Astrozyten, Neurone, Endothelzellen, Hepatocyten, pankreas-ähnliche Zellen und Lungen-Epithelzellen differenzieren. Morphologisch sind sie an ihrem fibroblastoiden Phänotyp zu erkennen, und sind in verschiedenen adulten und embryonalen Geweben des Menschen zu finden, darunter im Gehirn, Knochenmark, Nabelschnurblut, in Blutgefäßen, im Skelettmuskel, der Haut, Leber, Zahnfleisch und der Plazenta.

MSC aus Knochenmark exprimieren eine Reihe von Oberflächenmarkern wie bspw. CD105 (Endoglin, SH2), CD73 (Ecto-5'-Nucleotidase, SH3, SH4), CD166 (ALCAM), CD29 (β1-Integrin), CD44 (H-CAM) und CD90 (Thy-1), die z.T. auch auf endothelialen, epithelialen Zellen sowie auf Muskelzellen gefunden werden. MSC lassen sich aber von hämatopoetischen Stammzellen abgrenzen, da MSC die für hämatopoetische Stammzellen spezifischen Marker CD45, CD34 und CD133 nicht exprimieren.

MSC haben die Eigenschaft, schnell und stabil auf Plastik- oder Glasoberflächen zu adhärieren und Fibroblastenkolonien zu bilden ("colony forming units fibroblast" (CFU-F)). Letztere sind jedoch in Bezug auf ihre Proliferations- und Differenzierungsfähigkeiten heterogen.

MSC mit einem bestimmten Differenzierungspotential sind in der Medizin und Forschung von großem Interesse: Sie können insbesondere aus dem Knochenmark gewonnen werden, sogar aus jenem älterer Menschen, haben eine hohe Teilungsrate und können wie erwähnt in Gewebszellen mesenchymalen Ursprungs differenzieren. Sie könnten daher bspw. im Rahmen von Stammzelltherapien direkt der Behandlung degenerativer Erkrankungen von Organen wie Knochen, Knorpel, Sehnen, Muskel, Bindegewebe, Blutzellen, etc. dienen.

Zur Gewinnung bzw. Isolierung von MSC werden gegenwärtig unfraktionierte Knochenmarkszellen als Ausgangsmaterial verwendet. In Kulturflaschen adhärieren neben MSC auch andere Zellen wie Makrophagen und Endothelzellen. Nach definierten Zeitpunkten werden die nicht-adhärenten Zellen (meist hämatopoetische Zellen) aus der Probe verworfen. Die auf diese Weise gewonnenen Zellen sind jedoch wenig definiert und differenzieren sich nicht nur in heterogene MSC Populationen, sondern auch in Osteoblasten, und/oder in Osteoblasten-Vorläuferzellen, Fettzellen, Retikulumzellen, Makrophagen und Endothelzellen. Eine spezifische Behandlung degenerativer Erkrankungen eines Organs ist daher mit MSC ohne bestimmtes Differenzierungspotential schwierig bzw. auf Grund möglicher Nebenwirkungen problematisch.

Bühring et al. ("Phenotypic Characterization of Distinct Human Bone Marrow-Derived MSC Subsets", Hematopoietic Stern Cells VII, Ann. N.Y. Acad. Sci. 1176:124-134 (2009)) beschreiben zwei MSC-Subsets mit unterschiedlichem Expressionsmuster sowie unterschiedlicher Morphologie und Differenzierungsfähigkeit.

Die Isolierung von besonders reinen MSC, sowohl aus Primärgewebe als auch aus kultivierten MSC ist nach dem bisherigen Stand der Technik bisher nicht möglich oder bekannt, würde aber den Vorteil bieten, derart identifizierte Stammzellen gezielt für die Therapie/Behandlung von erkranktem, degeneriertem oder beschädigtem Gewebe einzusetzen, in welches sich die derart spezifisch isolierten Stammzellen differenzieren.

So könnten bspw. Knorpelschäden dadurch behandelt werden, dass spezifisch isolierte mesenchymale Stammzellen mit chondrogenem Differenzierungspotential entweder direkt *in situ* in das betroffene Gewebe gegeben werden, wo sie zu Chondrozyten ausdifferenzieren und dadurch das beschädigte Gewebe ersetzen (Stammzelltherapie). Andererseits kann aber auch eine Differenzierung in Chondrozyten *in vitro* von Interesse sein, wenn - bspw. für die Forschung/Diagnostik/Medizin - ausdifferenzierte Chondrozyten gewonnen werden sollen.

Vor diesem Hintergrund ist es Aufgabe der vorliegenden Erfindung, neue Wege bereitzustellen, mit denen besonders reine mesenchymale Stammzell-Populationen *in vitro* isoliert werden können.

Erfindungsgemäß wird diese Aufgabe gelöst durch die *in vitro* Verwendung von zumindest einem monoklonalen Antikörper, der an den Zelloberflächenmarker SUSD2 ("Sushi Domain containing protein 2" = Sushi Domäne enthaltendes Protein 2) bindet, oder funktionellen Fragmenten des Antikörpers, die an den Zelloberflächenmarker SUSD2 binden, in Kombination mit zumindest einem zweiten Antikörper, der an CD140b (PDGF-RB = Platelet derived growth factor receptor beta) bindet oder funktionellen Fragmenten des Antikörpers, sowie ggf. mit einem dritten Antikörper, der an TNAP ("tissue non-specific alkaline phosphatase" = Gewebe-unspezifische alkalische Phosphatase) und/oder CD56 bindet, oder funktionellen Fragmenten des Antikörpers. Durch den kombinierten *in vitro* Einsatz von diesen zumindest zwei Antikörpern wird die Signalstärke bei der Markierung erhöht, wodurch eine effektivere Reinigung bei der Selektion erzielt werden kann.

Die Aufgabe wird ferner gelöst durch ein *in vitro* Verfahren zur Isolierung und/oder Identifizierung von MSC, bei welchen die an SUSD2 und die an CD140b Antikörper, sowie ggf. die an TNAP und/oder CD56 bindenden Antikörper eingesetzt werden.

Die vorliegende Offenbarung beschreibt auch die auf diese Weise isolierten Stammzellen und deren Verwendung, insbesondere in der Therapie.

Die der Erfindung zugrunde liegende Aufgabe wird auf diese Weise vollkommen gelöst. Die Erfinder der vorliegenden Anmeldung konnten in eigenen Versuchen zeigen, dass es unter Verwendung der genannten Antikörper möglich ist, besonders reine MSC zu isolieren. Diese können anschließend, unter geeigneten *in vivo* oder *in vitro* Bedingungen, in Chondrozyten, Osteoblasten und Adipozyten differenzieren bzw. differenziert werden.

Damit wird zum ersten Mal ein Werkzeug bereitgestellt, mit dem hoch reine MSC-Populationen gewonnen werden können. Dies war bisher im Stand der Technik nicht möglich.

Insbesondere wird mittels des neu identifizierten Oberflächenmarkes SUSD2 ein Tool bereitgestellt, mit welchem erstmals - in Kombination mit CD140b, sowie ggf. zusätzlich mit CD56 und/oder TNAP - gezielt MSC identifiziert werden können, die eine besonders reine MSC-Subpopulation darstellen.

SUSD2 ist ein 822 Aminosäuren umfassendes integrales Membranprotein, das bisher in Zellen der Organe Mandeln, Lunge, Darm und Niere nachgewiesen wurde. Seine Sequenz und weitere Angaben finden sich in der UniProt Datenbank (www.uniprot.org) unter dem Eintrag Q9UGT4, auf die hiermit explizit Bezug genommen wird. Ein Zusammenhang mit der Expression dieses Membranproteins auf mesenchymalen Stammzellen wurde im Stand der Technik bisher nicht dargelegt.

Aufgrund der neuen *in vitro* Verwendung und des neuen *in vitro* Verfahrens können also MSC bereitgestellt werden, die bspw. wiederum vorteilhafterweise in der Therapie und Prophylaxe oder aber in der Diagnostik und Forschung eingesetzt werden können. So können die derart isolierten Stammzellen insbesondere zur Behandlung von Krankheiten, die sich durch ein degeneriertes, verletztes oder beschädigtes Gewebe auszeichnen, eingesetzt werden, bspw. im Rahmen einer Stammzelltherapie: Die mittels des erfindungsgemäßen *in vitro* Verfahrens isolierten Stammzellen werden hierfür in das betroffene Gewebe transplantiert (bspw. auch im Zusammenhang mit bestimmten Implantaten), und differenzieren sich dort in das entsprechende Gewebe. Dadurch wird das degenerierte Gewebe regeneriert und wieder funktionsfähig.

Mittels der neuen *in vitro* Verwendung können die mesenchymalen Stammzellen sowohl aus Primärgeweben als auch aus kultivierten Zellen hochrein isoliert und identifiziert werden.

Erfindungsgemäß können also anti-SUSD2-Antikörper in Kombination mit anti-CD140b Antikörpern, , sowie diese Kombination zusätzlich mit anti-TNAP-Antikörpern und/oder anti-CD56-Antikörpern, in der erfindungsgemäßen *in vitro* Verwendung und dem erfindungsgemäßen *in vitro* Verfahren eingesetzt werden.

In einer bevorzugten Ausführungsform der Erfindung ist der zumindest eine an SUSD2-bindende Antikörper ausgewählt aus:
- den Antikörpern W5C5 oder W3D5 oder aus einer Mischung dieser beiden Antikörper, die jeweils von Hybridomzelllinien produziert werden, die gemäß dem Budapester Vertrag jeweils am 21. Februar 2007 unter den Hinterlegungsnummern DSM ACC2813 (W5C5) und DSM ACC2815 (W3D5) bei der Deutschen Sammlung für Zellkulturen und Mikroorganismen hinterlegt wurden
- funktionellen Fragmenten der Antikörpers W5C5 oder W3D5, die noch in der Lage sind, an jeweils das gleiche Epitop zu binden wie die vollständigen Antikörper W5C5 und W3D5, und
- monoklonalen Antikörpern, die jeweils an das gleiche Epitop binden wie die Antikörper W5C5 oder W3D5.

Insbesondere ist bevorzugt, wenn der Antikörper W5C5 und/oder der Antikörper W3D5 eingesetzt wird. Die beiden Antikörper W5C5 oder W3D5 oder aus beiden dieser Antikörper, die von Hybridomzelllinien produziert werden, die gemäß dem Budapester Vertrag jeweils am 21. Februar 2007 unter den Hinterlegungsnummern DSM ACC2813 (W5C5) und DSM ACC2815 (W3D5) bei der Deutschen Sammlung für Zellkulturen und Mikroorganismen hinterlegt wurden; die Verlängerung der Hinterlegung wurde beantragt. Vorteilhafterweise können auch funktionelle oder bindungsrelevante Fragmenten dieser Antikörper eingesetzt werden. Dabei und vorliegend wird unter "bindungsrelevanten" oder "funktionellen" Fragmenten der Antikörper jedes Fragment der Antikörper, oder eine hieraus abgeleitete Sequenz, verstanden, das noch in der Lage ist, an das gleiche Epitop zu binden wie jeweils der vollständige Antikörper.

Die Antikörper W5C5 und W3D5 binden an unterschiedliche Epitope von SUSD2; obgleich diese Antikörper im Stand der Technik verfügbar sind, war das Antigen, an das diese Antikörper binden, bisher nicht bekannt.

In einer Weiterbildung der erfindungsgemäßen Verwendung ist dabei bevorzugt wenn der anti-CD140b-Antikörper ausgewählt ist aus der Gruppe:
- dem Antikörper 28D4,
- funktionelle Fragmente des Antikörpers 28D4, und
- ein Antikörper, der an das gleiche Epitop bindet wie der Antikörper 28D4.

Verschiedene anti-CD140b Antikörper sind bekannt und sind bspw. erhältlich bei GenWay Biotec, San Diego, USA, RayBiotech Inc., Norcross, USA, AbD Serotec, Raleigh, USA, BD Biosciences, San Jose, USA, Millipore Upstate Biotechnology, USA, Biolegend, San Diego, USA, etc.

Der CD140b-spezifische Antikörper 28D4 ist bspw. bei der Firma BD Biosciences, USA, erhältlich, und hat sich in anderen Studien als Marker für MSC erwiesen. Allerdings war bisher nicht bekannt, dass mit einer Kombination von Antikörpern, die gegen SUSD2 und CD140b gerichtet sind, hochreine Subpopulationen mesenchymaler Stammzellen gewonnen werden können.

In einer Weiterbildung der erfindungsgemäßen Verwendung ist dabei bevorzugt wenn der anti-TNAP-Antikörper ausgewählt ist aus der Gruppe:
- der Antikörper W8B2, der von der bei der Deutschen Sammlung von Mikroorganismen und Zellkulturen hinterlegten Zelllinie mit der Nr. ACC2567 produziert wird,
- funktionelle Fragmente des Antikörpers W8B2, der von der bei der Deutschen Sammlung von Mikroorganismen und Zellkulturen hinterlegten Zelllinie mit der Nr. ACC2567 produziert wird, und
- ein Antikörper, der an das gleiche Antigen oder Epitop bindet wie der Antikörper W8B2.

Der TNAP-spezifische Antikörper W8B2 hat sich in anderen Studien als Marker für MSC erwiesen. Allerdings war bisher nicht bekannt, dass mit einer Kombination von Antikörpern, die gegen SUSD2 und TNAP gerichtet sind, hochreine Subpopulationen mesenchymaler Stammzellen gewonnen werden können. Die den Antikörper W8B2 produzierenden Zellen wurden am 14. August 2002 bei der Deutschen Sammlung für Mikroorganismen und Zellkulturen (DSMZ) gemäß dem Budapester Vertrag hinterlegt, wobei die Verlängerung ihrer Hinterlegung gesichert wurde.

In einer bevorzugten Ausführungsform ist der an CD56 bindende Antikörper ausgewählt aus der Gruppe:
der Antikörper 39D5, der von der bei der Deutschen Sammlung von Mikroorganismen und Zellkulturen hinterlegten Zelllinie mit der Nr. DSM ACC 2930 produziert wird,
- funktionelle Fragmente des Antikörpers 39D5, der von der bei der Deutschen Sammlung von Mikroorganismen und Zellkulturen hinterlegten Zelllinie mit der Nr. DSM ACC 2930 produziert wird, und
- ein Antikörper, der an das gleiche Epitop bindet wie der Antikörper 39D5, der von der bei der Deutschen Sammlung von Mikroorganismen und Zellkulturen hinterlegten Zelllinie mit der Nr. DSM ACC 2930 produziert wird.

In einer Ausführungsform der erfindungsgemäßen *in vitro* Verwendung ist bevorzugt, wenn zu der Kombination aus einem anti-SUSD2 und einem gegen CD140b, sowie ggf. zusätzlich einem gegen CD56 und/oder TNAP gerichteten Antikörper, weiter zusätzlich noch ein gegen CD271 gerichteter Antikörper, oder funktionelle Fragmente davon, eingesetzt wird.

Vorliegend sollen, wie bereits weiter oben ausgeführt, die Begriffe "funktionale Fragmente" oder "funktionelle Fragmente", wie sie in der Anmeldung verwendet werden, Substanzen bedeuten, die Teile/Abschnitte der offenbarten Antikörper darstellen und die immer noch die funktionellen Eigenschaften, insbesondere die Zell-Bindungseigenschaften der Antikörper zeigen und besitzen, von denen sie abgeleitet sind. Dabei können diese Fragmente entweder als solche oder aber in Kombination mit anderen Fragmenten eingesetzt werden; vorliegend sind auch modifizierte W5C5, W3D5, W8B2, 39D5 und 28D4 Antikörper offenbart, die für entsprechende Einsätze und Verwendungen beim Menschen angepasst, bspw. humanisiert wurden.

Erfindungsgemäß können somit folgenden Kombinationen zur *in vitro* Isolierung/Identifizierung hochreiner MSC eingesetzt werden: W5C5 und/oder W3D5 mit anti-CD140b-Antikörpern, insbesondere 28D4; W5C5 und/oder W3D5 mit anti-CD140b-Antikörpern, insbesondere 28D4, und mit anti-CD56-Antiköprern oder anti-TNAP-Antikörpern; oder W5C5 und/oder W3D5 mit anti-CD140b-Antikörpern, insbesondere 28D4, anti-CD56-Antikörpern, insbesondere 39D5 und anti-TNAP-Antikörpern, insbesondere W8B2.

Die für die Zwecke der vorliegenden Erfindung geeigneten Antikörper sind monoklonal, wobei weitere, gegen SUSD2, CD140b, TNAP oder CD56 gerichtete Antikörper unter Einsatz der Antikörper W5C5 und W3D5, 28D4, W8B2 und 39D5 gewonnen werden können. Eine Anleitung zur Gewinnung von monoklonalen Antikörpern veröffentlichten Köhler und Milstein ("Continuous cultures of fused cells secreting antibody of predefined specificity", Nature, (1975), 256:495-497).

Vorliegend sind aber auch Fragmente solcher Antikörper, wie bspw. Fab, F(ab)'₂ oder scFv Fragmente, und andere Fragmente wie CDR ("complementaritydetermining region"), hypervariable Region) als Antikörper im Sinne und Rahmen der vorliegenden Erfindung gemeint, so lange wie sie ihre Funktionalität, d.h. die spezifischen Bindungseigenschaften wie die "ganzen" Antikörper, von denen sie abgeleitet sind, besitzen. Solche Antikörper-Fragmente können bspw. auch rekombinant unter Verwendung im Stand der Technik bekannter Verfahren hergestellt werden.

Die Antikörper W5C5 und W3D5 und 28D4 können auch entsprechend humanisiert und auch für eine Stammzelltherapie eingesetzt werden.

Humanisierte Antikörper sind bspw. chimäre Antikörper, bei welchen die konstanten Regionen der tierischen Antikörper (bspw. von Maus- oder Kaninchen-Antikörpern) durch die entsprechenden Regionen menschlicher Antikörper, bspw. den Fc-Fragmenten, ersetzt wurden (Sharon et al., Nature, (1984), 309:364-367). Alternativ kann auch die CDR der tierischen Antikörper mit menschlichen Antikörpern verbunden werden, dieser Prozess wird Antikörper "Reshaping" genannt. In einem weiteren anderen Verfahren werden in transgenen Tieren menschliche Antikörper produziert.

Die Antikörper können, bspw. in humanisierter Form, oder funktionelle Fragmente davon, auf entsprechende implantierbare medizinische Vorrichtungen auf- oder eingebracht werden, und zusammen mit der Vorrichtung in den zu behandelnden Patienten an die zu behandelnden Stellen/Gewebedefekten implantiert werden. An den zu behandelnden Stellen werden dann über die Antikörper mesenchymale Stammzellen rekrutiert, die sich an dem Implantat festsetzen, ausdifferenzieren und so neues Gewebe bilden. Geeignete medizinische Vorrichtungen sind hierbei irgendwelche biokompatiblen Implantate, Endoprothesen, bspw. Stents, etc., jeglicher Art, die entweder dauerhaft oder temporär in den Patienten eingebracht werden. Die Vorrichtungen können optional auch aus vollständig oder teilweise resorbierbaren Materialien bestehen und neben den Antikörpern weitere therapeutische Aktive Substanzen aufweisen, die üblicherweise bei in einen Körper einzubringenden Implantaten/Transplantaten eingesetzt werden.

Wie bereits weiter oben erwähnt, betrifft die vorliegende Erfindung auch ein *in vitro* Verfahren zur Isolierung und/oder Identifizierung von mesenchymalen Stammzellen, das die folgenden Schritte aufweist:
a) in-Kontakt-Bringen einer Probe, die mesenchymale Stammzellen enthält, mit zumindest einem Antikörper, der an Antigen SUSD2 bindet, oder mit funktionellen Fragmenten des Antikörpers,
b) ggf. in-Kontakt-Bringen einer Probe, die mesenchymale Stammzellen enthält, mit einem weiteren Antikörper, der an das Antigen SUSD2 bindet, oder mit funktionellen Fragmenten des Antikörpers,
c) in-Kontakt-Bringen der Probe mit zumindest einem Antikörper, der an CD140b bindet, und ggf. einem Antikörper, der an CD56 und/oder TNAP und/oder CD271 bindet, oder mit funktionellen Fragmenten der Antikörpers, und/oder
d) Isolieren und/oder Identifizieren von Zellen, an welche i) der zumindest eine Antikörper oder Fragmente davon, der an das Antigen SUSD2 bindet, und ii) der zumindest einen Antikörper, der an CD140b bindet, sowie ggf. iii) der zumindest eine Antikörper, der an CD56, TNAP und/oder CD271 bindet, gebunden hat.

Mit dieser Ausführungsform des erfindungsgemäßen *in vitro* Verfahrens können hochreine mesennchymale Stammzellen identifiziert und/oder isoliert werden, und insbesondere solche, die ein chondrogenes Differenzierungspotential besitzen. Die derart gewonnen Stammzellen können dann entweder direkt im Rahmen einer Stammzelltherapie (autologe oder allogene Therapie) eingesetzt werden, wo sie *in situ* in Chondrozyten differenzieren, und somit degeneriertes oder beschädigtes Knorpelgewebe regenerieren können.

Andererseits können die mit dem erfindungsgemäßen *in vitro* Verfahren gewonnenen MSC auch zunächst *in vitro* zu Chondrozyten differenziert werden, und anschließend zur Behandlung von erkranktem oder degeneriertem Gewebe eingesetzt werden.

Insbesondere in den letzten Jahren hat sich die autologe Chondrozyten Transplantation zu einem bevorzugten Eingriff zur Behandlung von (Gelenk-)Knorpeldefekten von Bandscheibe und Knie entwickelt, bei welchen der hyaline Knorpel wiederhergestellt werden soll. Hierzu werden dem Patienten durch eine Arthroskopie aus einem nicht beschädigten Gelenkanteil Proben entnommen, und die darin enthaltenen Knorpelzellen im Labor auf speziellen Matrices gezüchtet. Das dadurch entstehende Gewebe, also der neue Knorpel, wird dann durch eine gewebeschonende zweite Operation in das erkrankte/degenerierte Gelenk transplantiert.

Mit dem erfindungsgemäßen *in vitro* Verfahren ist es nun zum ersten Mal möglich, gezielt Stammzellen mit bspw. chondrogenem Differenzierungspotential aus dem Gewebe eines Patienten zu isolieren und eine schnelle, effiziente und gezielte Anzüchtung von Chondrozyten-Gewebe für die nachfolgende Transplantation in den Proben-Spender (autologe Transplantation) oder einen anderen Empfänger (allogene Transplantation) zu erreichen.

Bei den erfindungsgemäßen Verfahren ist dabei bevorzugt, wenn der anti-SUSD2-Antikörper wie weiter oben ausgeführt ausgewählt ist aus der Gruppe:
- den Antikörpern W5C5 oder W3D5 oder aus einer Mischung dieser Antikörper, die jeweils von Hybridomzelllinien produziert werden, die gemäß dem Budapester Vertrag jeweils am 21. Februar 2007 unter den Hinterlegungsnummern DSM ACC2813 (W5C5) und DSM ACC2815 (W3D5) bei der Deutschen Sammlung für Zellkulturen und Mikroorganismen hinterlegt wurden
- funktionellen Fragmenten der Antikörpers W5C5 oder W3D5, die noch in der Lage sind, an jeweils das gleiche Epitop zu binden wie die vollständigen Antikörper W5C5 und W3D5, und
- monoklonalen Antikörpern, die jeweils an das gleiche Epitop binden wie die Antikörper W5C5 oder W3D5.

Ferner ist bei dem erfindungsgemäßen Verfahren bevorzugt, wenn der anti-CD140b-Antikörper ausgewählt ist aus der Gruppe:
- der Antikörper 28D4,
- funktionelle Fragmente des Antikörpers 28D4, und
- Antikörper, die an das gleiche Epitop binden wie der Antikörper 28D4.

Ferner ist in einer Weiterbildung der erfindungsgemäßen Verfahren bevorzugt, wenn der an CD56 bindende Antikörper ausgewählt ist aus der Gruppe:
- der Antikörper 39D5, der von der bei der Deutschen Sammlung von Mikroorganismen und Zellkulturen hinterlegten Hybridomzelllinie mit der Nr. DSM ACC 2930 produziert wird,
- funktionelle Fragmente des Antikörpers 39D5, und
- Antikörper, die an das gleiche Antigen oder Epitop binden wie der Antikörper 39D5, der von der bei der Deutschen Sammlung von Mikroorganismen und Zellkulturen hinterlegten Hybridomzelllinie produziert wird.

Wie weiter oben für die erfindungsgemäße *in vitro* Verwendung erläutert, kann bei dem erfindungsgemäßen *in vitro* Verfahren einer oder mehrere anti-SUSD2-Antikörper eingesetzt werden, insbesondere die Antikörper W5C5 und W3D5 oder eine Kombination dieser beiden Antikörper, und zwar in Kombination mit einem anti-140b-Antikörper, oder ggf. in Kombination mit einer Mischung aus anti-140b, anti-CD56- und anti-TNAP-Antikörpern, wobei die Antikörper 28D4 (CD140b), 39D5 (CD56) und W8B2 (TNAP) bevorzugt sind.

Die Erfinder haben in eigenen Versuchen festgestellt, dass unter Verwendung der Antikörper W5C5 und/oder W3D5 in einem *in vitro* Verfahren zur Isolierung/Identifizierung von mesenchymalen Stammzellen eine gezielte Anreicherung von hochreinen mesenchymalen Stammzellen gewonnen werden konnte.

Im Rahmen der vorliegenden Erfindung wurde erkannt, dass die mit den erfindungsgemäßen *in vitro* Verfahren isolierten und/oder identifizierten Stammzellen für die Therapie, Diagnostik oder Forschung verwendet werden können.

Die mit den erfindungsgemäßen *in vitro* Verfahren gewonnenen Stammzellen können zur definierten Generierung von Chondrozyten eingesetzt werden, und zwar *in vivo* oder *in vitro.*

Die mit den erfindungsgemäßen *in vitro* Verfahren isolierten und/oder identifizierten Stammzellen, die zu Chondrozyten differenziert wurden, können auch zur Therapie und/oder Prophylaxe von degeneriertem oder anfälligem Gewebe eingesetzt werden.

Die mit den erfindungsgemäßen *in vitro* Verfahren gewonnenen Stammzellen können für die Therapie und/oder Prophylaxe von Knorpel- und/oder Knochenschäden, -degenerationen oder -erkrankungen, insbesondere der Knie und Bandscheiben, oder für rheumatoide Arthritis eingesetzt werden. Rheumatoide Arthritis stellt eine Autoimmunerkrankung dar, und auch bei dieser Krankheit kann die Verwendung von Stammzellen zum Gewebeersatz (also zum so genannten "tissue repair") zum Einsatz kommen.

Die vorliegende Beschreibung offenbart auch eine pharmazeutische Zusammensetzung sowie ein Kit, die/das eine Kombination von zumindest einem der Antikörper W5C5 oder W3D5 oder beide dieser Antikörper enthält, mit dem Antikörper 28D4 oder W8B2 oder 39D5.

Die vorliegende Beschreibung offenbart auch eine pharmazeutische Zusammensetzung, enthaltend Stammzellen, die gemäß den erfindungsgemäßen Verfahren isoliert und/oder identifiziert wurden, sowie zumindest einen pharmazeutisch akzeptierbaren Träger- und/oder Hilfsstoff, und ggf. therapeutisch wirksame Substanzen.

Unter "pharmazeutisch akzeptierbaren Träger- oder Hilfsstoffen" wird vorliegend jede in der Pharmazie im Zusammenhang mit an einen Patienten zu verabreichende Substanz/Zusammensetzung verstanden, die die Wirksamkeit der Zellen/Antikörper nicht nachteilig beeinflusst, und/oder die pharmakologisch die Anwendung der pharmazeutischen Zusammensetzung unterstützen oder erleichtern kann.

Unter "therapeutisch wirksamer Substanz" wird vorliegend jede Substanz verstanden, die für die Zwecke einer Behandlung oder Verbesserung eines Krankheitsbildes eines Patienten eingesetzt wird.

Die pharmazeutischen Zusammensetzungen können systemisch verabreicht werden, d.h. bspw. oral, subkutan, intravenös, rektal, parenteral, intramuskulär, intraperitoneal, transdermal, oder topisch, wobei die Verabreichungsart von der Art der Erkrankung, dem Krankheitsbild, sowie dem Zustand der Patienten abhängen wird. Ebenso kann die Verabreichung wiederholt oder einmalig stattfinden, wobei die Verabreichung im ersteren Fall einmal oder mehrmals am Tag, und/oder über einen längeren Zeitraum hinweg erfolgen kann.

Zusätzlich zu den aktiven Substanzen kann die pharmazeutische Zusammensetzung auch noch Puffer, Verdünnungsmittel und/oder Additive enthalten. Geeignete Puffer schließen bspw. Tris-HCI, Glycin und Phosphat mit ein, und geeignete Verdünnungsmittel bspw. wässrige NaCI-Lösungen, Lactose oder Mannitol. Geeignete Additive schließen bspw. Detergentien, Lösungsmittel, Antioxidantien und Konservierungsstoffe mit ein. Eine Übersicht über solche zusätzlichen Inhaltsstoffe findet sich bspw. in A. Kibbe.: "Handbook of Pharmaceutical Excipients", 3rd Ed., 2000, American Pharmaceutical Association and Pharmaceutical Press.

Die Erfindung wird in der nachstehenden Beschreibung und den beigefügten Figuren näher erläutert. In diesen zeigen:

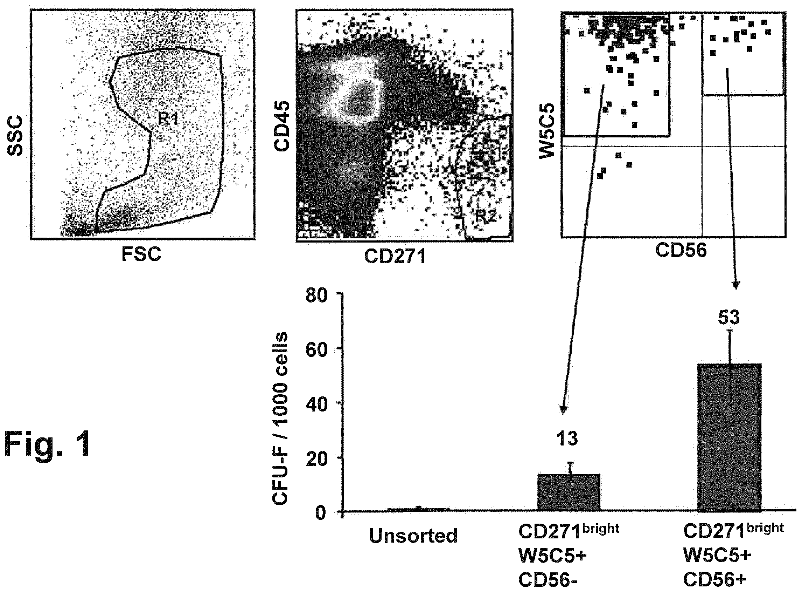

Fig. 1: CFU-F von SUSD2+ (W5C5) Knochenmarkszellen.
   Knochenmarkszellen wurden mit CD271-APC, CD45 Brilliant Violet, CD56-FITC und W5C5-PE markiert. Auf die CD271+CD45- MSC Population wurde ein Fenster gesetzt und anschließend im daraus resultierenden Plot Sortierfenster auf die W5C5+cd56- und die W5C5+CD56+ Populationen gesetzt. Nach der Sortierung wurde die Koloniebildungszahl (CFU-F) -Kapazität der jeweiligen Population getestet.

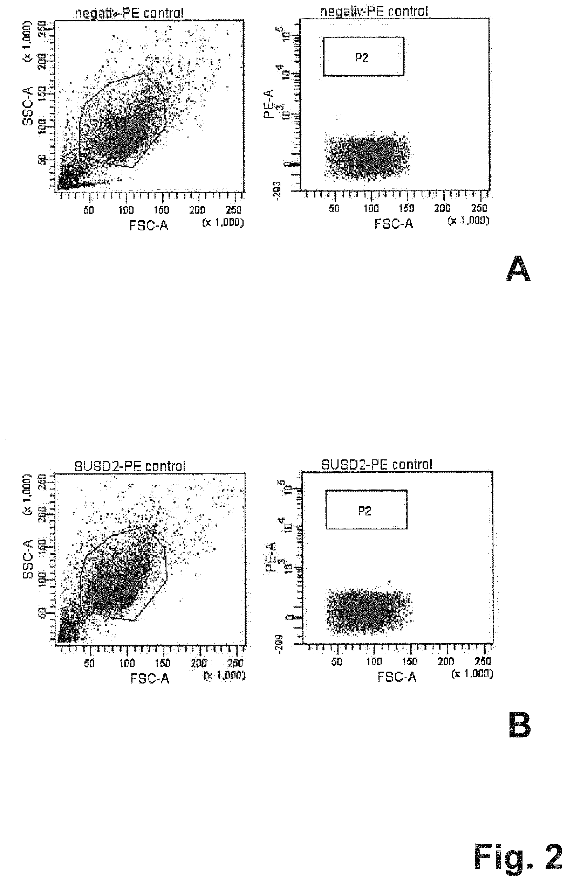

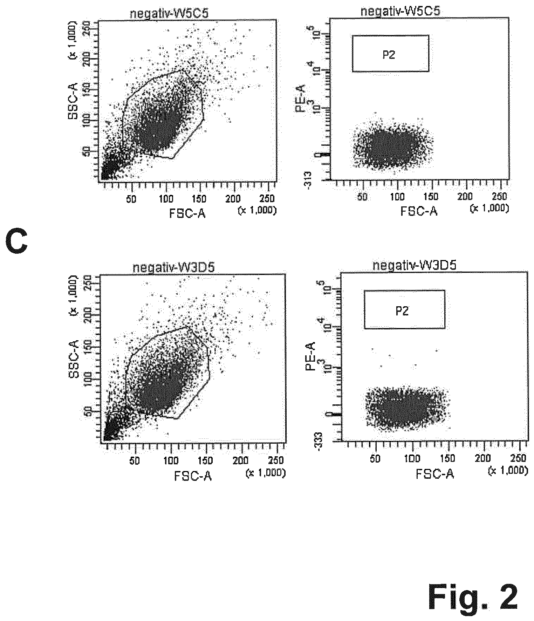

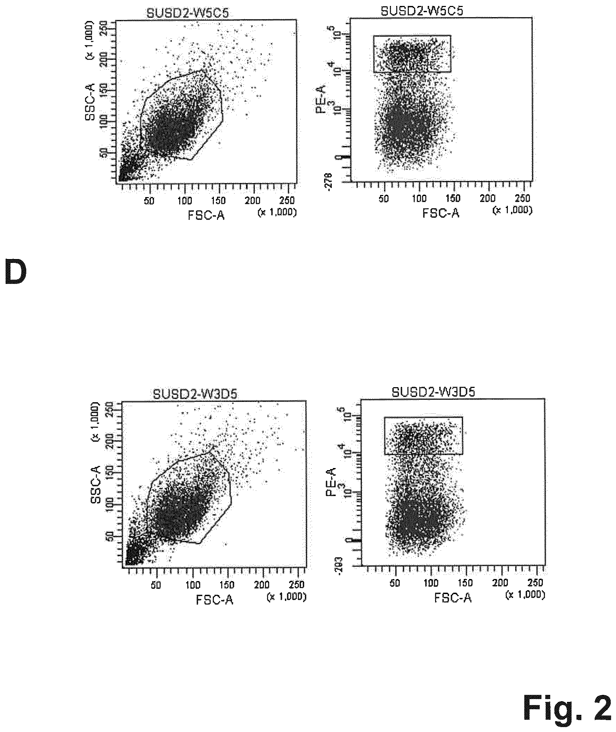

Fig. 2: SUSD2-Identifizierung:
   Die Antikörper W5C5 und W3D5 erkennen mit dem SUSD2-Gen transfizierte HEK-293 Zellen: Die FACS-Plots zeigen sowohl Kontrollfärbungen auf beiden Zelltypen (also nicht-transfizierte HEK-293 Zellen **(A)** und SUSD2-transfizierte HEK-293 Zellen **(B)** als auch spezifische Färbungen mit den jeweiligen Antikörpern (nicht-transfizierte HEK.293 Zellen **(C)** und SUSD2-transifizierte HEK293.Zellen **(D)**).

### Beispiele

### Material und Methoden

### Isolierung von Knochenmark- und peripheren blutmononuklearen Zellen

Nach Aufklärung und Einverständniserklärung wurde an der Berufsgenossenschaftlichen Klinik Knochenmark aus dem Oberschenkelschaft von Patienten gewonnen, die nach Hüftoperationen ein künstliches Hüftgelenk erhielten. Peripheres Blut von gesunden Spendern wurde von der Transfusionsabteilung des Universitätsklinikums Tübingen erhalten. Mononukleare Zellen aus dem Knochenmark und mononukleare Zellen aus dem Blut wurden mittels Ficoll-Dichtegradienten-Fraktionierung isoliert und die verbleibenden Erythrocyten in einer Ammoniumchloridlösung lysiert.

### Kultur der Primärzellen

Die Ficoll-getrennten und FACS-angereicherten Knochenmarkzellen wurden wie zuvor beschrieben kultiviert: 2 × 10⁷ unfraktionierte oder 1 × 10⁴ sortierte MSCA-1⁺CD56⁺ und MSCA-1⁺CD56⁻ BM-Zellen in Gelatine-beschichteten T-75 oder T-25 Kulturflaschen kultiviert, und zwar in Anwesenheit von 20 ml oder 6 ml Knockout™ Ersatzmedium (Invitrogen, Karlsruhe, Deutschland) sowie 5 ng/ml rekombinantem humanem Fibroblasten-Wachstumsfaktor (rh-bFGF; CellSystems, Remagen, Deutschland). Nach einer dreitägigen Kultur wurden die nicht-adhärierenden Zellen entfernt und frisches Medium hinzugefügt. Die adhärierenden Zellen wurden kultiviert, bis sie eine 90%-ige Konfluenz erreichten.

### Koloniebildendes Fibroblast-Assay (CFU-F)

CFU-F-Assays wurden durch Ausplattieren von entweder 1 × 10⁵ unselektierten oder 500 - 5.000 FACS-selektierten Knochenmarkzellen in Gelatine-beschichtete T-25 Flaschen durchgeführt, die Knockout™-Medium und 5 ng/ml rh-bFGF enthielten. Nach einer zwölftägigen Kultivierung wurden die adhärierenden Zellen zweimal mit PBS gewaschen, fünf Minuten bei Raumtemperatur mit Methanol fixiert (Sigma-Aldrich), luftgetrocknet und mit Giemsa-Lösung gefärbt (Merck, Darmstadt, Deutschland). CFU-F-Kolonien wurden makroskopisch ausgezählt. Die Größe der Kolonien reichte von zwischen 1 und 8 mm im Durchmesser.

### Immunfluoreszenzanalyse und Zellsortierung

*Antikörper.* Die folgenden Antikörper wurden eingesetzt: W5C5 (SUSD2), W3D5 (SUSD2), und W8B2 (TNAP). CD56-FITC (Klon NCAM16.2) wurde von Becton Dickinson (Heidelberg, Deutschland) gekauft. Der SSEA-4-reaktive Antikörper MC-813-70 wurde von Chemicon (Hampshire, Großbritannien) erworben. CD271-APC und SUSD2-PE und mit MACS beads gekoppeltem SUSD2 wurden von Miltenyi Boitec, Bergisch Gladbach, erworben.

*Immunofluoreszenzfärbung:* Nach Blockierung und spezifischen Bindungen mit 10 mg/ml Polyglobin (10 min, 4 °C) wurden die Zellen 15 min lang mit entweder 20 µl Antikörpern oder 10 µl Fluorochrom-konjugierten Antikörpern inkubiert. Die mit den Konjugaten gefärbten Zellen wurden zweimal gewaschen, in 200 µl FACS Puffer suspendiert und für die Durchflusszytometrie eingesetzt. Die Zellen, die mit den Antikörpern markiert waren, wurden mit 20 µl eines F(ab)₂-Fragment des R-Phycoerythrin (PE)-konjugierten Ziegen-anti-Maus-Antikörpers (Dako Cytomations, Glostrup, Dänemark) 15 min lang gefärbt, zweimal gewaschen und mittels Durchflusszytometrie analysiert. Für die Vielfarbenfärbung wurden die Zellen 15 min lang mit 10 µl eines Anti-CD56-FITC, Anti-TNAP-APC und anti-SUSD2-PE. Nach dem Waschen wurden die Zellen für die Durchflusszytometrie eingesetzt. Für eine kombinierte indirekte und direkte Färbung wurden die Zellen zunächst mit dem nicht-konjugierten Antikörper markiert, und anschließend mit 20 µl an 1:25 verdünntem Ziegen-anti-Maus sekundärem Antikörper 15 min lang gefärbt. Die freien Bindestellen des sekundären Antikörpers wurden durch Inkubation der Zellen für 25 min mit 20 µl an einem Maus-IgG-polyklonalen Antikörper (0,05 µg/ml; Southern Biotech, Birmingham, AL) blockiert, bevor sie mit TNAP-APC und/oder CD56-FITC gegengefärbt wurden. Nach einem Waschschritt wurden die Zellen mittels Durchflusszytometrie analysiert.

### Duchflusszytometrische Analysen und Zellsortierung

Knochenmarkzellen, die mit Anti-TNAP-APC, Anti-SUSD2-PE und CD56-FITC markiert wurden, wurden mit einem FACSAria Zellsortierer (Becton Dickinson) sortiert, indem zunächst ein Gate auf die SUSD2 Population gesetzt wurde und anschließend die Sortierfenster im resultierenden Plot TNAP gegen CD56 gesetzt wurden. Die sortierten Zellen wurden für funktionelle und phänotypische Analysen eingesetzt. Die Koexpressionsanalysen erfolgten mit einem FACSCantoll Durchflusszytometer (Becton Dickinson). Die Daten wurden unter Verwendung der FCS-Express-Software (De Novo Software, Ontario, Kanada) analysiert.

### MACS-Trennung

In ausgewählten Versuchen wurden die Knochenmarkzellen mittels MACS (Miltenyi Biotec) vorsortiert unter Verwendung von SUSD2-PE und Anti-PE MACS Beads. Die Trennungen wurden gemäß den Empfehlungen des Herstellers durchgeführt.

Wie bereits bei der Figurenbeschreibung erwähnt, wurden in Versuchen Knochenmarkzellen mit CD271-APC, CD45-BrilliantViolet, CD56-FITC und W5C5-PE markiert. Da MSC CD271⁺CD45⁻ sind, wurde auf diese Population ein Fenster gesetzt und anschließend im daraus resultierenden Plot Sortierfenster auf die W5C5⁺CD56⁻ und W5C5⁺CD56⁺ Populationen gesetzt. Nach der Sortierung wurde die Koloniebildungskapazität der jeweiligen Populationen untersucht (Fig. 1).

### SUSD2-Identifizierung

Kultivierte Knochenmark-MSC wurden simultan mit W3D5 und W5C5 markiert und mit einem Kaninchen-anti-Maus-PE-Konjugat gefärbt (durch Vorversuche mit über 20 verschiedenen Zelltypen wurde herausgefunden, dass W5C5 und W3D5 die gleichen Reaktivitätsmuster hatten). Anschließend wurden die W5C5IW3D5⁻ und W5C5/W3D5⁺⁺⁺ Zellen im FACS-Sorter sortiert und abzentrifugiert. Aus den Zellpellets wurde die mRNA isoliert (über Mitenyi, Deutschland) und eine Genexpressionsanalyse der beiden Fraktionen durchgeführt. Es zeigte sich, dass in der W5C5/W3D5⁺⁺⁺ Fraktion das SUSD2-Gen um ca. den Faktor 20 höher exprimiert war als in der W5C5/W3D5⁻ Fraktion. Da es keine monoklonalen SUSD2-Antikörper auf dem Markt gab, wurde die komplette Sequenz des SUSD2-Gens erworben (Origen) und damit HEK-293 Zellen transfiziert. 3 Tage nach der Transfektion wurde die Reaktivität von W5C5 und W3D5 auf der Transfektante und der nicht-transfizierten HEK-293 Zelle verglichen (Fig. 4). Die FACS-Plots zeigen sowohl Kontrollfärbungen auf beiden Zelltypen (Fig. 4A, B) als auch spezifische Färbungen mit den jeweiligen Antikörpern (Fig. 4C, D). Es stellte sich heraus, dass beide Antikörper selektiv die HEK293/huSUSD2 Transfektante erkennen.

Mit den vorliegenden Ergebnissen konnte somit gezeigt werden, dass die Kombination von zumindest den Antikörpern gegen SUSD2 und CD140b,TNAP und/oder CD56 geeignet ist, alle MSC aus Knochenmark hochrein und effektiv zu isolieren, insbesondere deshalb, da die beiden Marker SUSD2 hoch-selektiv für MSC sind. TNAP ist stark auf CD56⁻ MSC aber nur schwach auf CD56⁺ MSC exprimiert. SUSD2 ist jedoch auf beiden Subsets gleich stark exprimiert. Die Kombination von beiden Antikörpern ermöglicht durch die Signalverstärkung eine sichere Selektion von MSC.

Mit der vorliegenden Studie wurde daher eine Kombination aus Antigenen identifiziert, nämlich SUSD2 mit CD104b, TNAP und/oder CD56, über welche effektiv MSC, insbesondere solche mit chondrogenem, adipozytärem und osteogenem Differenzierungspotential, isoliert und/oder identifiziert werden können.

Diese Ergebnisse sind insbesondere im Hinblick auf den klinischen Einsatz derart isolierter Stammzellen bzw. der über diese Stammzellen gewonnenen Chondrozyten, Adipozyten und Osteoblasten relevant. So sind bspw. Verletzungen des Gelenkknorpels und von Bandscheiben regelmäßig schwierig zu behandeln, gerade aufgrund der begrenzten Regenerationsfähigkeit dieser Gewebe. Krankheiten wie rheumatoide Arthritis, Traumata, Knochenbrüche und Bandscheibenverletzungen sind direkt mit dem Mangel einer effektiven Chondrogenese verbunden. Trotz des Fortschritts in der Orthopädie und des steigenden Erfolgs bei der autologen Chondrozytentransplantation bleiben Zellbiologie-bezogene Ansätze für die Knorpelregeneration eine Herausforderung. Das Hauptproblem ist die Verwendung kultivierter Zellen für klinische Zwecke, bei welchen die Ausgangszellen lediglich schlecht charakterisiert sind.

Daher bietet die vorliegende Erfindung die Möglichkeit, bspw. hoch angereicherte und gut definierte MSC Knochenmarkszellen bereitzustellen, mit einer herausragenden chondrogenen Differenzierungsfähigkeit, die für den klinischen Einsatz als Ausgangspopulation eingesetzt werden können. Diese Zellen können entweder direkt zur Injektion eingesetzt werden, bspw. in die Bandscheibenzwischenräume/Bandscheiben, oder *in vitro* expandiert und in Chondrozyten differenziert werden, bevor sie für klinische Einsätze verwendet werden.

## Patentansprüche

1. Verwendung von zumindest einem ersten Antikörper, der an das Antigen Sushi Domäne enthaltendes Protein 2 (SUSD2) bindet, oder funktionellen Fragmenten des zumindest ersten Antikörpers, in Kombination mit zumindest einem zweiten Antikörper, der an das Antigen CD140b bindet, oder funktionellen Fragmenten des zumindest zweiten Antikörpers, zur *in vitro* Isolierung von mesenchymalen Stammzellen.

2. Verwendung nach Anspruch 1, **dadurch gekennzeichnet, dass** der zumindest eine an SUSD2 bindende Antikörper ausgewählt ist aus der Gruppe:
- der Antikörper W5C5, der von der bei der Deutschen Sammlung von Mikroorganismen und Zellkulturen hinterlegten Zelllinie mit der Nr. ACC 2813 produziert wird,
- der Antikörper W3D5, der von der bei der Deutschen Sammlung von Mikroorganismen und Zellkulturen hinterlegten Zelllinie mit der Nr. ACC2815 produziert wird,
- funktionelle Fragmente der Antikörper W5C5 oder W3D5, und
- ein Antikörper, der an das gleiche Epitop bindet wie der Antikörper W5C5 oder W3D5.

3. Verwendung nach Anspruch 1 oder 2, **dadurch gekennzeichnet, dass** der an CD140b bindende Antikörper ausgewählt ist aus der Gruppe:
- der Antikörper 28D4
- funktionelle Fragmente des Antikörpers 28D4, und
- ein Antikörper, der an das gleiche Epitop bindet wie der Antikörper 28D4.

4. Verwendung nach einem der Ansprüche 1 bis 3, **dadurch gekennzeichnet, dass** die Verwendung in Kombination mit zumindest einem dritten Antikörper, oder funktionellen Fragmenten davon, erfolgt, der zumindest an eines der Antigene CD56 oder TNAP (tissue non-specific alkaline phosphatase; Gewebe-unspezifische alkalische Phosphatase) bindet, wobei der an TNAP bindende Antikörper ausgewählt ist aus der Gruppe:
der Antikörper W8B2, der von der bei der Deutschen Sammlung von Mikroorganismen und Zellkulturen hinterlegten Zelllinie mit der Nr. ACC2813 produziert wird,
- funktionelle Fragmente des Antikörpers W8B2, und
- ein Antikörper, der an das gleiche Epitop bindet wie der Antikörper W8B2.

5. Verwendung nach Anspruch 4, **dadurch gekennzeichnet, dass** der an CD56 bindende Antikörper ausgewählt ist aus der Gruppe:
- der Antikörper 39D5, der von der bei der Deutschen Sammlung von Mikroorganismen und Zellkulturen hinterlegten Zelllinie mit der Nr. ACC2930 produziert wird,
- funktionelle Fragmente des Antikörpers 39D5, und
- ein Antikörper, der an das gleiche Epitop bindet wie der Antikörper 39D5.

6. *In vitro* Verfahren zur Isolierung und/oder Identifizierung von mesenchymalen Stammzellen, insbesondere von mesenchymalen Stammzellen mit chondrozytärem differenzierungspotential, **dadurch gekennzeichnet, dass** es die folgenden Schritte aufweist:
a) in-Kontakt-Bringen einer Probe, die mesenchymale Stammzellen enthält, mit zumindest einem Antikörper, der an Antigen SUSD2 bindet, oder mit funktionellen Fragmenten des Antikörpers,
b) ggf. in-Kontakt-Bringen einer Probe, die mesenchymale Stammzellen enthält, mit einem weiteren Antikörper, der an das Antigen SUSD2 bindet, oder mit funktionellen Fragmenten des Antikörpers,
c) in-Kontakt-Bringen der Probe mit zumindest einem Antikörper, der an CD140b bindet, oder mit funktionellen Fragmenten des Antikörpers, und
d) Isolieren und/oder Identifizieren von Zellen, an welche i) der zumindest eine Antikörper oder Fragmente davon, der an das Antigen SUSD2 bindet, und ii) der zumindest eine Antikörper, der an CD140b bindet, gebunden hat.

7. Verfahren nach Anspruch 6, **dadurch gekennzeichnet, dass** der in Schritt a) eingesetzte anti-SUSD2-Antikörper ausgewählt ist aus der Gruppe:
- der Antikörper W5C5, der von der bei der Deutschen Sammlung von Mikroorganismen und Zellkulturen hinterlegten Hybridomzelllinie mit der Nr. ACC2813 produziert wird,
- der Antikörper W3D5, der von der bei der Deutschen Sammlung von Mikroorganismen und Zellkulturen hinterlegten Hybridomzelllinie mit der Nr. ACC2815 produziert wird,
- funktionelle Fragmente der Antikörper W5C5 und W3D5, und Antikörper, die an das gleiche Epitop binden wie der Antikörper W5C5 oder W3D5.

8. Verfahren nach einem der Ansprüche 6 oder 7, **dadurch gekennzeichnet, dass** der in Schritt c) eingesetzte anti-CD140b-Antikörper ausgewählt ist aus der Gruppe:
der Antikörper 28D4,
- funktionelle Fragmente des Antikörpers 28D4, und
- Antikörper, die an das gleiche Epitop binden wie der Antikörper 28D4.

9. Verfahren nach einem der Ansprüche 6 bis 8, **dadurch gekennzeichnet, dass** es den weiteren Schritt c1) aufweist:
c1) in-Kontakt-Bringen der Probe mit zumindest einem Antikörper, der an CD56
oder TNAP bindet, oder mit funktionellen Fragmenten des Antikörpers, wobei der in Schritt c1) eingesetzte anti-TNAP-Antikörper ausgewählt ist aus der Gruppe:
- der Antikörper W8B2, der von der bei der Deutschen Sammlung von Mikroorganismen und Zellkulturen hinterlegten Hybridomzelllinie mit der Nr. ACC2567 produziert wird,
- funktionelle Fragmente des Antikörpers W8B2, und
- Antikörper, die an das gleiche Antigen oder Epitop binden wie der Antikörper W8B2.

10. Verfahren nach Anspruch 9, **dadurch gekennzeichnet, dass** der in Schritt c1) eingesetzte an CD56 bindende Antikörper ausgewählt ist aus der Gruppe:
- der Antikörper 39D5, der von der bei der Deutschen Sammlung von Mikroorganismen und Zellkulturen hinterlegten Hybridomzelllinie mit der Nr. ACC 2930 produziert wird,
- funktionelle Fragmente des Antikörpers 39D5, und
- Antikörper, die an das gleiche Epitop binden wie der Antikörper 39D5.

11. Verfahren nach einem der Ansprüche 6 bis 10, **dadurch gekennzeichnet, dass** die Schritte a), b), c) und ggf. c1) gleichzeitig, aufeinanderfolgend oder in umgekehrter Reihenfolge durchgeführt werden.

## Claims

1. Use of at least one first antibody which binds to the antigen Sushi domain containing protein 2 (SUSD2), or functional fragments of this at least one first antibody, in combination with at least one second antibody which binds to the antigen CD140b, or functional fragments of the at least second antibody, for the *in vitro* isolation of mesenchymal stem cells.

2. Use as claimed in claim 1, **characterized in that** the at least one antibody binding to SUSD2 is selected from the group:
- the antibody W5C5 which is produced by the cell line deposited at the German Collection of Microorganisms and Cell Cultures with the No. ACC 2813,
- the antibody W3D5 which is produced by the cell line deposited at the German Collection of Microorganisms and Cell Cultures with the No. ACC 2815,
- functional fragments of the antibodies W5C5 or W3D5, and
- an antibody which binds to the same epitope as the antibodies W5C5 or W3D5.

3. Use as claimed in claim 1 or 2, **characterized in that** the antibody binding to CD140b is selected from the group:
- the antibody 28D4,
- functional fragments of the antibody 28D4, and
- an antibody which binds to the same epitope as the antibody 28D4.

4. Use as claimed in one of claims 1 to 3, **characterized in that** the use is carried out in combination with at least one third antibody, or functional fragments thereof, which binds to at least one of the antigens CD56 or TNAP (tissue non-specific alkaline phosphatase), wherein the antibody binding to TNAP is selected from the group:
- the antibody W8B2 which is produced by the cell line deposited at the German Collection of Microorganisms and Cell Cultures with the No. ACC2813
- functional fragments of the antibody W8B2, and
- an antibody which binds to the same epitope as the antibody W8B2.

5. Use as claimed in claim 4, **characterized in that** the antibody binding to CD56 is selected from the group:
- the antibody 39D5 which is produced by the cell line deposited at the German Collection of Microorganisms and Cell Cultures with the No. ACC2930
- functional fragments of the antibody 39D5, and
- an antibody which binds to the same epitope as the antibody 39D5.

6. *In vitro* method for the isolation and/or identification of mesenchymal stem cells, in particular of mesenchymal stem cells with chondrocyte differentiation potential, **characterized in that** it comprises the following steps:
a) contacting a sample which contains mesenchymal stem cells with at least one antibody which binds to the antigen SUSD2, or with functional fragments of the antibody,
b) optionally contacting a sample which contains mesenchymal stem cells with a further antibody which binds to the antigen SUSD2, or with functional fragments of the antibody,
c) contacting the sample with at least one antibody which binds to CD140b, or with functional fragments of the antibody, and
d) isolation and/or identification of cells to which i) the at least one antibody or fragments thereof which binds to the antigen SUSD2 and ii) the at least one antibody which binds to CD140b has bound.

7. The method as claimed in claim 6, **characterized in that** the anti-SUSD2 antibody used in step a) is selected from the group:
- the antibody W5C5 which is produced by the cell line deposited at the German Collection of Microorganisms and Cell Cultures with the No. ACC2813,
- the antibody W3D5 which is produced by the cell line deposited at the German Collection of Microorganisms and Cell Cultures with the No. ACC2815,
- functional fragments of the antibodies W5C5 and W3D5, and
- antibodies which bind to the same epitope as the antibodies W5C5 or W3D5.

8. The method as claimed in one of claims 6 or 7, **characterized in that** the anti-CD140b-antibody used in step c) is selected from the group:
- the antibody 28D4,
- functional fragments of the antibody 28D4, and
- antibodies which bind to the same epitope as the antibody 28D4.

9. The method as claimed in one of claims 6 to 8, **characterized in that** it comprises the additional step c1),
c1) contacting the sample with at least one antibody which binds to CD56 or TNAP, or with functional fragments of the antibody,
wherein the anti-TNAP-antibody used in step c1) is selected from the group:
- the antibody W8B2 which is produced by the cell line deposited at the German Collection of Microorganisms and Cell Cultures with the No. ACC2567
- functional fragments of the antibody W8B2, and
- antibodies which bind to the same epitope as the antibody W8B2.

10. The method as claimed in claim 9, **characterized in that** the antibody used in step c1) and binding to CD56 is selected from the group:
- the antibody 39D5 which is produced by the cell line deposited at the German Collection of Microorganisms and Cell Cultures with the No. ACC2930,
- functional fragments of the antibody 39D5, and
- antibodies which bind to the same epitope as the antibody 39D5.

11. The method as claimed in one of claims 6 to 10, **characterized in that** the steps a), b), c) and, as the case may be, c1), are performed simultaneously, successively, or in reversed order.

## Revendications

1. Utilisation d'au moins un premier anticorps, qui se lie à l'antigène de la protéine 2 contenant des domaines Sushi (SUSD2), ou de fragments fonctionnels dudit au moins un premier anticorps, en combinaison avec au moins un deuxième anticorps, qui se lie à l'antigène CD140b, ou des fragments fonctionnels dudit au moins un deuxième anticorps, pour l'isolement *in vitro* de cellules souches mésenchymateuses.

2. Utilisation selon la revendication 1, **caractérisée en ce que** ledit au moins un anticorps qui se lie à SUSD2 est choisi dans le groupe constitué par :
- l'anticorps W5C5, qui est produit par la lignée cellulaire déposée sous le numéro ACC 2813 auprès de la collection allemande de micro-organismes et de cultures cellulaires,
- l'anticorps W3D5, qui est produit par la lignée cellulaire déposée sous le numéro ACC 2815 auprès de la collection allemande de micro-organismes et de cultures cellulaires,
- des fragments fonctionnels de l'anticorps W5C5 ou W3D5, et
- un anticorps qui se lie au même épitope que l'anticorps W5C5 ou W3D5.

3. Utilisation selon la revendication 1 ou 2, **caractérisée en ce que** l'anticorps qui se lie à CD140b est choisi dans le groupe constitué par :
- l'anticorps 28D4,
- des fragments fonctionnels de l'anticorps 28D4, et
- un anticorps qui se lie au même épitope que l'anticorps 28D4.

4. Utilisation selon l'une quelconque des revendications 1 à 3, **caractérisée en ce que** l'utilisation a lieu en combinaison avec au moins un troisième anticorps, ou des fragments fonctionnels de celui-ci, qui se lie au moins à un des antigènes CD56 ou TNAP (tissue non-specific alkaline phosphatase ; phosphatase alcaline non spécifique au tissu), l'anticorps qui se lie à TNAP étant choisi dans le groupe constitué par :
- l'anticorps W8B2, qui est produit par la lignée cellulaire déposée sous le numéro ACC 2813 auprès de la collection allemande de micro-organismes et de cultures cellulaires,
- des fragments fonctionnels de l'anticorps W8B2, et
- un anticorps qui se lie au même épitope que l'anticorps W8B2.

5. Utilisation selon la revendication 4, **caractérisée en ce que** l'anticorps qui se lie à CD56 est choisi dans le groupe constitué par :
- l'anticorps 39D5, qui est produit par la lignée cellulaire déposée sous le numéro ACC 2930 auprès de la collection allemande de micro-organismes et de cultures cellulaires,
- des fragments fonctionnels de l'anticorps 39D5, et
- un anticorps qui se lie au même épitope que l'anticorps 39D5.

6. Procédé *in vitro* pour l'isolement et/ou l'identification de cellules souches mésenchymateuses, notamment de cellules souches mésenchymateuses à potentiel de différenciation chondrocytaire, **caractérisé en ce qu'**il comprend les étapes suivantes :
a) la mise en contact d'un échantillon qui contient des cellules souches mésenchymateuses avec au moins un anticorps qui se lie à l'antigène SUSD2, ou avec des fragments fonctionnels de l'anticorps,
b) éventuellement la mise en contact d'un échantillon qui contient des cellules souches mésenchymateuses avec un autre anticorps qui se lie à l'antigène SUSD2, ou avec des fragments fonctionnels de l'anticorps,
c) la mise en contact de l'échantillon avec au moins un anticorps qui se lie à CD140b, ou avec des fragments fonctionnels de l'anticorps, et
d) l'isolement et/ou l'identification de cellules auxquelles i) ledit au moins un anticorps ou des fragments de celui-ci, qui se lie à l'antigène SUSD2, et ii) ledit au moins un anticorps qui se lie à CD140b se sont reliés.

7. Procédé selon la revendication 6, **caractérisé en ce que** l'anticorps anti-SUSD2 utilisé à l'étape a) est choisi dans le groupe constitué par :
- l'anticorps W5C5, qui est produit par la lignée cellulaire d'hybridomes déposée sous le numéro ACC 2813 auprès de la collection allemande de micro-organismes et de cultures cellulaires,
- l'anticorps W3D5, qui est produit par la lignée cellulaire d'hybridomes déposée sous le numéro ACC 2815 auprès de la collection allemande de micro-organismes et de cultures cellulaires,
- des fragments fonctionnels des anticorps W5C5 et W3D5, et
- un anticorps qui se lie au même épitope que l'anticorps W5C5 ou W3D5.

8. Procédé selon l'une quelconque des revendications 6 ou 7, **caractérisé en ce que** l'anticorps anti-CD140b utilisé à l'étape c) est choisi dans le groupe constitué par :
- l'anticorps 28D4,
- des fragments fonctionnels de l'anticorps 28D4, et
- un anticorps qui se lie au même épitope que l'anticorps 28D4.

9. Procédé selon l'une quelconque des revendications 6 à 8, **caractérisé en ce qu'**il comprend l'étape c1) supplémentaire :
c1) la mise en contact de l'échantillon avec au moins un anticorps qui se lie à CD56 ou TNAP, ou avec des fragments fonctionnels de l'anticorps,
l'anticorps anti-TNAP utilisé à l'étape c1) étant choisi dans le groupe constitué par :
- l'anticorps W8B2, qui est produit par la lignée cellulaire d'hybridomes déposée sous le numéro ACC 2567 auprès de la collection allemande de micro-organismes et de cultures cellulaires,
- des fragments fonctionnels de l'anticorps W8B2, et
- un anticorps qui se lie au même antigène ou épitope que l'anticorps W8B2.

10. Procédé selon la revendication 9, **caractérisé en ce que** l'anticorps qui se lie à CD56 utilisé à l'étape c1) est choisi dans le groupe constitué par :
- l'anticorps 39D5, qui est produit par la lignée cellulaire d'hybridomes déposée sous le numéro ACC 2930 auprès de la collection allemande de micro-organismes et de cultures cellulaires,
- des fragments fonctionnels de l'anticorps 39D5, et
- un anticorps qui se lie au même épitope que l'anticorps 39D5.

11. Procédé selon l'une quelconque des revendications 6 à 10, **caractérisé en ce que** les étapes a), b), c) et éventuellement c1) sont réalisées simultanément, successivement ou dans l'ordre inverse.
